# EUROPÄISCHE PATENTSCHRIFT

(11) **EP 2 768 438 B1**
(45) Veröffentlichungstag und Bekanntmachung des Hinweises auf die Patenterteilung: **29.03.2017**
(21) Anmeldenummer: 12783513.0
(22) Anmeldetag: 17.10.2012
(51) Int. Cl.: A61F 2/78, A61F 2/30

(54) **VERBINDUNGSSYSTEM UND PROTHESENSYSTEM**
CONNECTING SYSTEM AND PROSTHESIS SYSTEM
SYSTÈME DE LIAISON ET SYSTÈME DE PROTHÈSE

(30) Priorität: 19.10.2011 DE 102011116280
(43) Veröffentlichungstag der Anmeldung: 27.08.2014
(73) Patentinhaber: Otto Bock HealthCare GmbH, 37115 Duderstadt (DE)
(72) Erfinder: HILLMANN, Martin, 37115 Duderstadt (DE)
(74) Vertreter: Lins, Edgar
(86) Internationale Anmeldenummer: PCT/EP2012/004340
(87) Internationale Veröffentlichungsnummer: WO 2013/056824

(56) Entgegenhaltungen:
- EP-B1- 1 435 880
- WO-A1-2006/076011
- WO-A2-03/039398
- GB-A- 2 338 899
- US-B2- 6 589 288
- US-B2- 6 979 354

## Beschreibung

Die Erfindung betrifft ein Verbindungssystem eingerichtet zum lösbaren Verbinden eines Prothesenschaftes mit einem über einen Amputationsstumpf gezogenen Liner, das einen Verbindungsstift und eine Aufnahmeeinrichtung mit einer Aufnahme umfasst, in die der Verbindungsstift einführbar ist. Die Erfindung betrifft zudem ein Prothesensystem mit einem Liner und einem Prothesenschaft, wobei das Prothesensystem ein derartiges Verbindungssystem umfasst.

Ein gattungsgemäßes Verbindungssystem ist beispielsweise aus der US 6,979,354 B2 und der WO 2006/076011 A1 bekannt.

Um eine Prothese, beispielsweise eine Unterschenkelprothese, an einem

Amputationsstumpf sicher zu befestigen, wird über diesen Amputationsstumpf zunächst ein Liner gezogen, der beispielsweise aus einem Silikonmaterial bestehen kann. Am distalen Ende des Liners befindet sich ein Verbindungsstift, der auf unterschiedliche Weisen mit dem Liner verbunden sein kann.

Am Prothesenschaft hingegen befindet sich eine Aufnahmeeinrichtung, die eine Aufnahme umfasst, in die dieser Verbindungsstift einführbar ist. Zum Verbinden des Prothesenschaftes mit dem Liner wird einfach der Verbindungsstift in die dafür vorgesehene Aufnahme eingeführt. Dabei weist der Stift umlaufende Nuten auf, mit denen er in ein Ritzel bzw. Zahnrad eingreifen kann, das sich in der Aufnahmeeinrichtung befindet. Durch die besondere Ausgestaltung dieser Aufnahmeeinrichtung kann der Stift auf diese Weise nur in die Aufnahme eingeführt werden, ohne jedoch einfach wieder aus ihr herausgezogen werden zu können. Dazu muss beispielsweise ein Druckmechanismus betätigt werden, durch den das Ritzel bzw. Zahnrad mit den Nuten des Verbindungsstiftes außer Eingriff gebracht wird.

Nachteilig ist, dass der Liner vom Patienten nicht jedes Mal in identischer Weise über den Amputationsstumpf gezogen werden kann, so dass auch der an dem Liner befindliche Verbindungsstift in leicht unterschiedlichen Orientierungen am Amputationsstumpf angeordnet wird. Dadurch ist es möglich, dass der Verbindungsstift die falsche Orientierung aufweist, um ihn in die Aufnahme der Aufnahmeeinrichtung des Prothesenschaftes einführen zu können.

Daher ist es aus der US 6,589,288 B2 bekannt, den Verbindungsstift an seinem dem Amputationsstumpf zugewandten Ende mit einer Kugel auszustatten, die wie ein Kugelgelenk mit der Befestigungsanordnung, über die der Verbindungsstift am Liner festgelegt wird, zusammenwirkt. Dadurch ist eine Verschwenkung und eine laterale Verschiebung des Verbindungsstiftes in engen Grenzen möglich.

Nachteilig ist, dass insbesondere durch die laterale Verschieblichkeit des Stiftes nur sehr geringe Abweichungen von der optimalen Position des Verbindungsstiftes am Amputationsstumpf ausgeglichen werden können. Durch die Verschwenkbarkeit hingegen ist es möglich, dass der Verbindungsstift auch dann in die Aufnahme der Aufnahmeeinrichtung eingebracht werden kann, wenn er sich deutlicher von seiner optimalen Position weg befindet. Beim Einführen des Verbindungsstiftes in die Aufnahme wird daher der Verbindungsstift so zur Seite verschwenkt, dass die distale Spitze, also das dem Amputationsstumpf abgewandte Ende des Verbindungsstiftes, in die Aufnahme hineinragt. Dadurch, dass der Verbindungsstift in diesem Fall jedoch eine deutliche Winkelabweichung von seiner optimalen Lage aufweist, ist es möglich, dass er nicht vollständig in die Aufnahme eingeführt werden kann, was zu einer nicht optimalen Befestigung des Prothesenschaftes am Amputationsstumpf führt. Dadurch können wunde Stellen und Schmerzen sowie Fehlbelastungen und Fehlstellungen beim Patienten auftreten.

Um diesem Problem zu begegnen ist es aus der EP 1 435 880 B1 bekannt, den Verbindungsstift vollständig flexibel auszugestalten. Er wird beispielsweise aus einem synthetischen Material oder als Schraubenfeder geformt und ist in jeder mit seiner Längsachse zusammenfallenden Ebene flexibel und auch in axialer Richtung elastisch flexibel ausgebildet. Damit kann ein derartiger Verbindungsstift bis zu einem gewissen Grad jegliche Fehlstellung und Fehlpositionierung des Verbindungsstiftes relativ zur Aufnahme der Aufnahmeeinrichtung ausgleichen. Nachteilig ist jedoch, dass durch die dafür erforderliche in allen Richtungen wirkende Flexibilität und Elastizität des Verbindungsstiftes dessen Druckstabilität deutlich reduziert wird. Insbesondere beim Einführen des Verbindungsstiftes in die Aufnahme der Aufnahmeeinrichtung beim Anlegen des Prothesensystems kann es daher dazu kommen, dass der Verbindungsstift nach außen ausknickt, anstatt weiter in die Aufnahme eingeführt zu werden. Dies führt zu einer nur sehr schlechten Befestigung des Prothesenschaftes am Liner und zudem zu falschen Druckbelastungen und gegebenenfalls Druckstellen am Amputationsstumpf des Patienten.

Der Erfindung liegt daher die Aufgabe zugrunde, ein Verbindungssystem vorzuschlagen, mit dem leichte Fehlstellungen und Fehlpositionierungen des Liners und damit des Verbindungsstiftes relativ zum Prothesenschaft ausgeglichen werden können, und dennoch die erforderliche Sicherheit gewährleistet wird, die Prothese sicher und fehlerfrei anlegen zu können.

Die Erfindung löst die gestellte Aufgabe durch ein gattungsgemäßes Verbindungssystem, dass sich dadurch auszeichnet, dass der Verbindungsstift wenigstens zwei starre Stiftsegmente umfasst, die entlang einer Längsrichtung des Verbindungsstiftes, also von dessen ersten Ende zu dessen zweiten Ende, gegeneinander verlagerbar hintereinander angeordnet sind.

Durch die Verwendung wenigstens zweier starrer Stiftsegmente, die selbst also nicht flexibel oder elastisch ausgebildet sind, ist es möglich, die erforderliche Druckstabilität, die insbesondere beim Einführen des Verbindungsstiftes in die Aufnahme der Aufnahmeeinrichtung benötigt wird, zu gewährleisten. Da die einzelnen Stiftsegmente jedoch gegeneinander verlagerbar, also insbesondere gegeneinander verkipp- und verschwenkbar sind, ist die ausreichende Flexibilität gegeben, Fehlstellungen und Fehlpositionierungen des Verbindungsstiftes relativ zur Aufnahmeeinrichtung ausgleichen zu können. Um die Stiftsegmente derart zu lagern, kann es auch nötig sein, sie gegeneinander verschiebbar zu lagern.

Insbesondere die Verwendung von genau zwei starren Stiftsegmenten hat sich als vorteilhaft erwiesen. Bei der Verwendung nur eines starren Stiftsegmentes, ist keine Verschwenkung gegeneinander möglich. Eine derartige Lösung ist aus dem Stand der Technik bekannt und führt zu dem genannten Problem. Werden mehr als zwei starre Stiftsegmente verwendet, erhöht sich naturgemäß auch die Anzahl der Stellen, an denen zwei benachbarte Stiftsegmente gegeneinander verschwenkt werden können. Insbesondere bei einem nicht exakten Einführen des Verbindungsstiftes in die Aufnahme der Aufnahmeeinrichtung kann es in dieser Ausgestaltung dazu kommen, dass zumindest ein Teil des Verbindungsstiftes nach außen ausbricht. So ist es beispielsweise möglich, dass das Stiftsegment, welches als erstes in die Aufnahme eingeführt wird, in der Aufnahme enthalten ist, während das zweite und dritte Stiftsegment nach außen herausgeschwenkt sind und so ein tieferes Einführen des Verbindungsstiftes in die Aufnahme verhindern. Dies führt zu einer nur sehr unsicheren Verbindung des Prothesenschaftes mit dem Liner. Dennoch ist auch in dieser Ausgestaltung eine exakte und in vielen Orientierungen flexibel verwendbare Verbindung möglich.

In einer bevorzugten Ausführungsform sind die einzelnen Stiftsegmente auf ein flexibles Führungselement aufgefädelt. Dieses kann beispielsweise ein Seilelement, beispielsweise ein Stahlseil oder ein Kunststoffseil sein. Die einzelnen Stiftsegmente können wie Perlen auf einer Kette auf diesen Führungselement aufgefädelt sein, so dass sie gegeneinander verlagerbar, insbesondere verkippbar und verschwenkbar, sind.

Vorzugsweise weist der Verbindungsstift ein erstes Ende zum Anordnen an dem Liner und ein dem ersten Ende entgegengesetztes zweites Ende auf, und die Stiftsegmente sind durch ein Spannelement in Richtung auf das zweite Ende vorgespannt. Dabei kann das Spannelement beispielsweise eine Feder, insbesondere eine Schraubenfeder, oder ein Elastomer, beispielsweise in Form eines Schlauches, umfassen. Bevorzugt ist das Spannelement in Form eines elastischen Schlauches ausgebildet. Das Spannelement ist in einer besonders einfachen Ausführungsform ebenfalls auf das flexible Führungselement aufgefädelt.

Durch das Spannelement ist es möglich, die wenigstens zwei Stiftsegmente, die auf das Führungselement aufgefädelt sind, gegeneinander zu verschwenken oder zu verkippen.

An dem ersten Ende des Verbindungsstiftes, das dem Liner zugewandt ist, befindet sich vorteilhafterweise ein Verbindungselement zum Verbinden des Verbindungsstiftes mit dem Liner. Dies kann beispielsweise ein Außengewinde sein, so dass das Verbindungselement in ein dafür vorgesehenes korrespondierendes Gewinde am Liner eingeschraubt wird. Natürlich sind auch alle anderen Möglichkeiten, den Verbindungsstift am Liner festzulegen, denkbar.

Besonders bevorzugt ist das Spannelement zwischen den Stiftsegmenten und dem ersten Ende des Verbindungsstiftes angeordnet. Damit befindet sich das Spannelement näher am Liner als die einzelnen Stiftsegmente.

Vorzugsweise weisen die Stiftsegmente eine Mehrzahl von um die Längsrichtung umlaufenden Nuten auf, die ausgebildet sind, um in ein in der Aufnahmeeinrichtung vorgesehenes Ritzel oder ein Zahnrad oder eine ähnliche Vorrichtung einzugreifen, wobei das Ritzel beziehungsweise das Zahnrad oder die Vorrichtung in einer Einführposition, in der der Verbindungsstift in die Aufnahmeeinrichtung einführbar ist, und in eine Freigabeposition bringbar ist, in der der Verbindungsstift aus der Aufnahmeeinrichtung entfernbar ist.

Derartige Anordnungen, bei denen ein Ritzel in eine Einführposition und eine Freigabeposition bringbar ist, sind aus dem Stand der Technik bekannt. Zusammen mit der besonderen Anordnung der Stiftsegmente und dem Spannelement entlang des Führungselementes bilden sich jedoch mehrere Vorteile gegenüber den aus dem Stand der Technik bekannten Lösungen.

Beim Anlegen der Prothese wird der Verbindungsstift, der an dem Liner angeordnet ist, in die Aufnahme der Aufnahmeeinrichtung eingeführt. Die einzelnen Nuten der Stiftsemente greifen in das Ritzel ein, wenn der Verbindungsstift in die Aufnahme eingeführt wird. Ein seitliches Ausknicken wird durch die verwendeten starren Segmente wirksam verhindert.

Beim Gehen mit der Prothese wird der Stift beim Auftreten mit der Prothese druckbelastet. Dabei wird er folglich weiter in Richtung auf die Aufnahme der Aufnahmeeinrichtung gedrückt und dringt gegebenenfalls tiefer in diese Aufnahme ein. In dieser Phase des Gehens wird folglich ein optimaler Sitz und Halt des Verbindungsstiftes in der Aufnahme erreicht. In der Schwungphase eines jeden Schrittes wird der Verbindungsstift zugbelastet. Da der Verbindungsstift jedoch entlang seiner Längsachse nicht elastisch ausgebildet ist, kommt es hier nicht zu einer Relativbewegung zwischen dem Prothesenschaft und dem Liner. Damit wird ein Scheuern oder Reiben des Prothesenschaftes am Liner und damit am Amputationsstumpf des Patienten vermieden, wodurch der Tragekomfort deutlich erhöht wird.

Vorteilhafterweise weist die Aufnahmeeinrichtung eine Einführeinrichtung auf, durch die die Orientierung und/oder die Positionierung des Verbindungsstiftes in der Aufnahme vereinfacht werden. Dies kann beispielsweise ein trichterförmig ausgestaltetes Element sein, durch das das zweite Ende des Verbindungsstiftes in die Aufnahme der Aufnahmeeinrichtung geführt wird, sobald es sich innerhalb des Trichters befindet.

Alternativ oder zusätzlich dazu kann die Einführeinrichtung wenigstens ein magnetisches Element umfassen und der Verbindungsstift zumindest teilweise aus einem Material bestehen, auf das durch das magnetische Element eine Kraft ausgeübt wird, durch die der Verbindungsstift auf die Aufnahme ausgerichtet wird. So ist es beispielsweise denkbar, in der Nähe der Aufnahme einen Permanentmagneten an der Aufnahmeeinrichtung anzuordnen und auch am Verbindungsstift, vorzugsweise in der Nähe seines zweiten Endes, einen Permanentmagneten anzuordnen. Beide sollten dabei so ausgerichtet sein, dass ungleichnamige Pole aufeinander ausgerichtet sind. Dadurch wird eine Anziehungskraft hervorgerufen, durch die der Verbindungsstift besonders einfach in die Aufnahme der Aufnahmeeinrichtung eingeleitet wird. Natürlich ist es bei geeigneter Materialwahl ausreichend, nur einen Permanentmagneten vorzusehen. Alternativ oder zusätzlich dazu kann auch ein ringförmiges Magnetelement an der Aufnahmeeinrichtung vorgesehen sein, das um die Aufnahme herumgelegt ist. Ist nun beispielsweise das zweite Ende des Verbindungsstiftes aus einem Material, auf das durch dieses magnetische Element eine Kraft ausgeübt wird, kommt es zu einer Zentrierung des Verbindungsstiftes und insbesondere dessen zweiten Endes auf die eigentliche Aufnahme hin. Dadurch wird die Einführung des Verbindungsstiftes in die Aufnahme weiter vereinfacht.

Ein erfindungsgemäßes Prothesensystem mit einem Liner und einem Prothesenschaft umfasst zudem ein hier beschriebenes Verbindungssystem, wobei der Verbindungsstift an dem Liner und die Aufnahmeeinrichtung an dem Prothesenschaft angeordnet sind.

Mit Hilfe einer Zeichnung wird nachfolgend ein Ausführungsbeispiel der vorliegenden Erfindung näher erläutert. Es zeigt
- Figur 1: - die schematische Ansicht eines Verbindungsstiftes für ein Verbindungssystem gemäß einem ersten Ausführungsbeispiel der vorliegenden Erfindung,
- Figur 2: - eine schematische Schnittdarstellung durch den Verbindungsstift aus Figur 1,
- Figur 3: - den Verbindungsstift aus den Figuren 1 und 2 in einer Explosionsdarstellung,
- Figur 4: - eine schematische Schnittdarstellung durch ein Verbindungssystem gemäß einem weiteren Ausführungsbeispiel der vorliegenden Erfindung,
- Figur 5: - ein Verbindungssystem gemäß einem Ausführungsbeispiel der vorliegenden Erfindung in einer Explosionsdarstellung,
- Figur 6: - die schematische Ansicht eines Verbindungsstiftes für ein Verbindungssystem gemäß einem weiteren Ausführungsbeispiel der vorliegenden Erfindung,
- Figur 7: - eine schematische Schnittdarstellung durch den Verbindungsstift aus Figur 6,
- Figur 8: - den Verbindungsstift aus den Figuren 6 und 7 in einer Explosionsdarstellung,
- Figur 9: - eine Explosionsdarstellung eines Verbindungsstiftes für ein Verbindungssystem gemäß einem weiteren Ausführungsbeispiel der vorliegenden Erfindung,
- Figur 10: - eine schematische Schnittdarstellung durch ein Verbindungssystem gemäß einem weiteren Ausführungsbeispiel der vorliegenden Erfindung,
- Figur 11: - eine Explosionsdarstellung eines Prothesensystems gemäß einem Ausführungsbeispiel der vorliegenden Erfindung und
- Figur 12: - eine schematische Schnittdarstellung durch das Prothesensystem aus Figur 11.

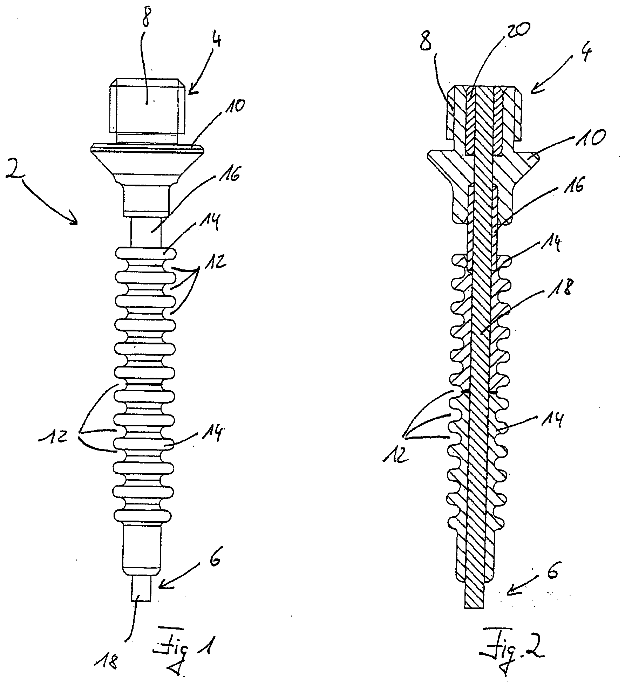

Figur 1 zeigt die schematische Ansicht eines Verbindungsstiftes 2 für ein Verbindungssystem gemäß einem ersten Ausführungsbeispiel der vorliegenden Erfindung.

Der Verbindungsstift 2 verfügt über ein erstes Ende 4 zum Anordnen an einem Liner sowie ein zweites Ende 6 mit dem der Verbindungsstift in einer Aufnahme einer Aufnahmeeinrichtung einführbar ist. An dem ersten Ende 4 ist ein Verbindungselement 8 dargestellt, mit dem der Verbindungsstift 2 am Liner festgelegt werden kann. Dies kann beispielsweise in Form einer Schraubverbindung geschehen. In diesem Fall verfügt das Verbindungselement 8 beispielsweise über ein Außengewinde.

In Figur 1 unterhalb des Verbindungselementes 8 befindet sich ein Anschlag 10, mit dem der Verbindungsstift 2 am Liner anliegt, wenn er über das Verbindungselement 8 mit dem Liner verbunden ist.

Der Verbindungsstift 2 verfügt über seine Länge über eine Mehrzahl von in seine Längsrichtung umlaufenden Nuten 12. Diese Nuten sind an zwei Stiftsegmenten 14 angeordnet, die entlang einer Längsrichtung des Verbindungsstiftes 2 hintereinander angeordnet sind. Zwischen dem ersten Ende 4 und den beiden Stiftsegmenten 14 befindet sich ein Spannelement 16, das im gezeigten Ausführungsbeispiel als ein elastischer Schlauch ausgebildet ist. Alternativ dazu kann das Spannelement 16 beispielsweise auch in Form einer Schraubenfeder ausgebildet sein.

Sowohl das Spannelement 16 als auch die Stiftelemente 14 sind auf ein flexibles Führungselement 18 aufgefädelt, von dem in Figur 1 nur am zweiten Ende 6 ein kleiner Abschnitt zu sehen ist.

Figur 2 zeigt die Darstellung aus Figur 1 in einer Schnittdarstellung. Man erkennt, dass das flexible Führungselement 18 vom ersten Ende 4 bis zum zweiten Ende 6 des Verbindungsstiftes 2 durchläuft. Alle übrigen Bauteile sind auf dieses flexible Führungselement 18 aufgefädelt und an diesem befestigt. In Figur 2 ist deutlich besser zu erkennen, dass die Nuten 12 an zwei wie in Figur 2 übereinander dargestellten Stiftsegmenten 14 angeordnet sind.

Wird der Verbindungsstift 2 nun in eine Aufnahme eingeführt, zu der er nicht optimal positioniert ist, kann diese Fehlpositionierung dadurch ausgeglichen werden, dass die beiden Stiftsegmente 14 gegeneinander verlagerbar angeordnet sind. Dazu wird beispielsweise das flexible Führungselement 18 gebogen, so dass die darauf angeordneten Stiftsegmente 14 leicht auseinander geschoben werden. Dabei wird das Spannelement 16 in diesem Fall gewölbt oder, wenn es als Schraubenfeder ausgebildet ist, leicht zusammen geschoben. Dadurch wird die Flexibilität des Verbindungsstiftes 2 gewährleistet, ohne zuviel der nötigen Druckstabilität aufzugeben. Durch die besondere Ausgestaltung sind die Stiftsegmente 14 in alle Richtungen gegeneinander verschwenkbar und verkippbar, ohne dass die Gesamtlänge des Verbindungsstiftes 2, also von seinem ersten Ende 4 zu seinem zweiten Ende 6, verändert wird. Dadurch wird eine Relativbewegung zwischen dem Liner und dem Prothesenschaft zumindest stark eingeschränkt, im Optimalfall sogar vollständig verhindert.

Das flexible Führungselement 18 ist am ersten Ende 4 über eine Festlegung 20 befestigt. Dies kann beispielsweise eine Schraub- oder Klebeverbindung oder eine Löt- oder Schweißverbindung sein. Dadurch wird die Gesamtlänge, also der Abstand vom ersten Ende 4 zum zweiten Ende 6 des Verbindungsstiftes 2 festgelegt. Er ist in dieser Form nicht entlang seiner Längsachse elastisch ausgebildet. Dadurch kommt es auch beim Gehen mit einer Prothese, die durch einen derartigen Verbindungsstift am Liner festgehalten wird, nicht zu einer Relativbewegung zwischen dem Prothesenschaft und dem Liner, so dass es auch nicht zu Wunden durch Scheuern oder falsche Druckbelastung am Amputationsstumpf des Patienten kommen kann.

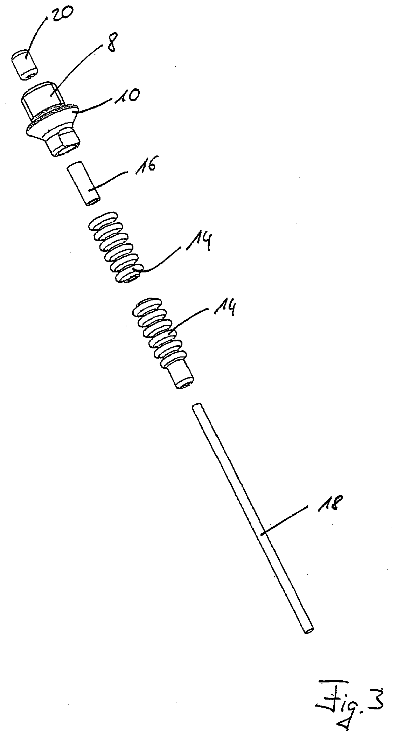

Figur 3 zeigt den Verbindungsstift 2 aus den Figuren 1 und 2 in einer Explosionsdarstellung. Die beiden Stiftsegmente 14 werden auf das flexible Führungselement 18 geschoben. Im Bereich des ersten Endes 4, in Figur 3 also im oberen Ende, des Verbindungsstiftes 2 befindet sich das Spannelement 16 sowie das Verbindungselement 8 und der Anschlag 10, wobei das flexible Führungselement 18 am Verbindungselement 8 über die Festlegung 20 festgelegt ist.

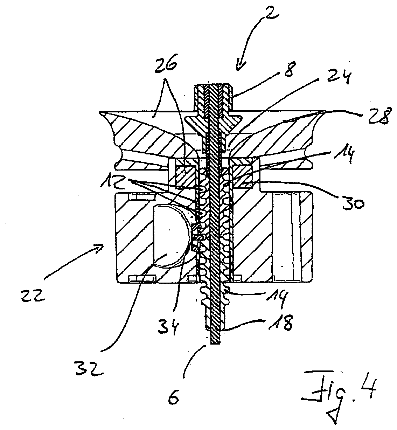

Figur 4 zeigt eine Schnittdarstellung durch ein Verbindungssystem gemäß einem Ausführungsbeispiel der vorliegenden Erfindung. Zentral befindet sich der Verbindungsstift 2 mit seinem Verbindungselement 8 am zweiten Ende 6. Entlang seines flexiblen Führungselementes 18 befinden sich die zwei Stiftsegmente 14. Natürlich sind auch Ausführungsformen mit mehr als zwei, beispielsweise drei, vier oder fünf Stiftsegmenten 14 denkbar. Je nach gewünschter Länge des Verbindungsstiftes 2 können auch andere Anzahlen von Stiftsegmenten 14 verwendet werden.

Um den Verbindungsstift 2 herum ist eine Aufnahmeeinrichtung 22 dargestellt. Diese weist im oberen Abschnitt eine Aufnahme 24 auf, in die der Verbindungsstift 2 eingeführt ist. Die Aufnahme 24 verfügt dabei über eine Einführeinrichtung 26, die in Figur 4 aus zwei Elementen besteht. Zum einen ist um die Aufnahme 24 herum die Aufnahmeeinrichtung 22 in Form eines Trichters 28 ausgebildet. Zum anderen ist um die eigentliche Aufnahme 24 herum ein magnetisches Element 30 angeordnet, das die Aufnahme 24 und damit im in Figur 4 gezeigten Zustand auch den Verbindungsstift ringförmig umfasst. Alternativ oder zusätzlich dazu kann ein weiteres magnetisches Element 30 vorgesehen sein, das in Figur 4 jedoch nicht dargestellt ist. Dabei kann es sich beispielsweise um ein zylinderförmiges magnetisches Element handeln, durch das auf den Verbindungsstift 2 eine Kraft ausgeübt wird, die das zweite Ende 6 des Verbindungsstiftes 2 in die Aufnahme 24 hineinzieht.

Ein Bauteil des Verbindungsstiftes 2 ist aus einem Material hergestellt, auf das durch das magnetische Element 30 eine Kraft ausgeübt wird. In der gezeigten Ausführungsform wird so beim Einführen des Verbindungsstiftes 2 das zweite Ende 6 in Richtung auf die Aufnahme 24 zentriert.

Im unteren Teil der Aufnahmeeinrichtung 22 ist ein Ritzel 32 schematisch dargestellt. Das Ritzel 32 verfügt über Zähne 34, die in die Nuten 12 der Stiftsegmente 14 eingreifen. Im in Figur 4 gezeigten Zustand befindet sich das Ritzel 32 in der Einführposition.

In dieser Position ist es möglich, den Verbindungsstift im in Figur 4 gezeigten Ausführungsbeispiel von oben in die Aufnahmeeinrichtung 22 einzuführen. Die Zähne 34 des Ritzels 32 greifen in die Nut 12 ein und das Ritzel 32 ist ausgebildet, sich in der dafür nötigen Richtung drehen zu können. Eine Drehung des Ritzels 32 in der entgegengesetzten Richtung jedoch ist nicht möglich. Dadurch wird verhindert, dass der Verbindungsstift 2 beispielsweise bei der Schwungphase eines Gehschrittes einfach wieder aus der Aufnahmeeinrichtung 22 herausgezogen wird.

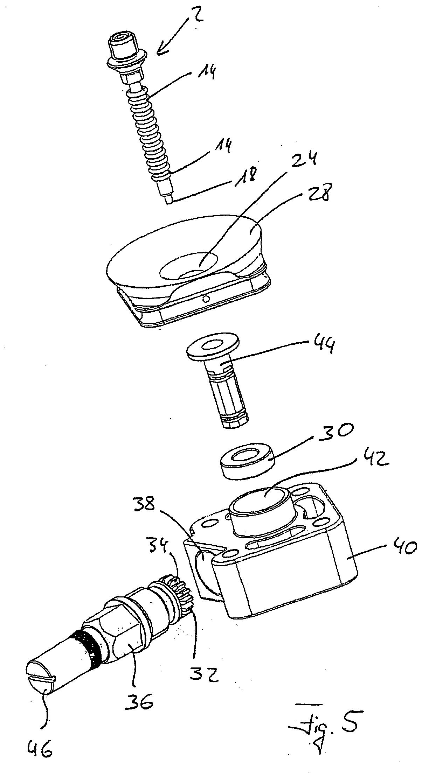

Figur 5 zeigt das in Figur 4 gezeigte Verbindungssystem in einer Explosionsdarstellung. Das Ritzel 32 mit den darin befindlichen Zähnen 34 ist an einer Rasteinheit 36 angeordnet, die in eine dafür vorgesehene Öffnung 38 einer Verriegelungsplatte 40 eingeführt wird. Senkrecht zu der Öffnung 38 weist die Verriegelungsplatte 40 eine Zentralbohrung 42 auf, in die sowohl das magnetische Element 30 als auch eine Einpressbuchse 44 eingeführt wird. Die Einpressbuchse 44 endet im oberen Bereich mit der Aufnahme 24, in die der Verbindungsstift 2 eingeführt wird.

Befindet sich das Ritzel 32 in der Einführposition, greifen seine Zähne 34 in die Nuten 12 der Stiftsegmente 14 ein. Am dem Ritzel 32 abgewandten Ende der Rasteinheit 36 ist ein Bedienelement 46 dargestellt, durch das die Rasteinheit 36 um ihre Längsachse gedreht werden kann. Dazu muss in den in der Bedieneinheit 46 vorgesehenen Schlitz beispielsweise ein Schraubenzieher, eine Münze oder ein ähnliches geeignetes Werkzeug eingebracht werden. Durch Drehung dieses Werkzeuges lässt sich die gesamte Rasteinheit 36 drehen. Dies ist jedoch nur in einer Richtung möglich. Durch das Drehen in diese eine Richtung wird der Verbindungsstift 2 noch weiter in die Aufnahme 24 der Aufnahmeeinrichtung 22 hineingezogen. Eine Drehung in die andere Richtung wird verhindert, um auszuschließen, dass der Verbindungsstift 2 aus der Aufnahme 24 der Aufnahmeeinrichtung 22 herausbefördert wird.

Soll das Ritzel 32 von der Einführposition in die Freigabeposition gebracht werden, ist es beispielsweise denkbar, über ein Drücken des Bedienelementes 46 in Richtung auf die Verriegelungsplatte 40, das Ritzel 32 mit den Nuten 12 der Stiftsegmente 14 außer Eingriff zu bringen. In diesem Fall lässt sich der Verbindungsstift 2 einfach nach oben aus der Aufnahme 24 herausziehen.

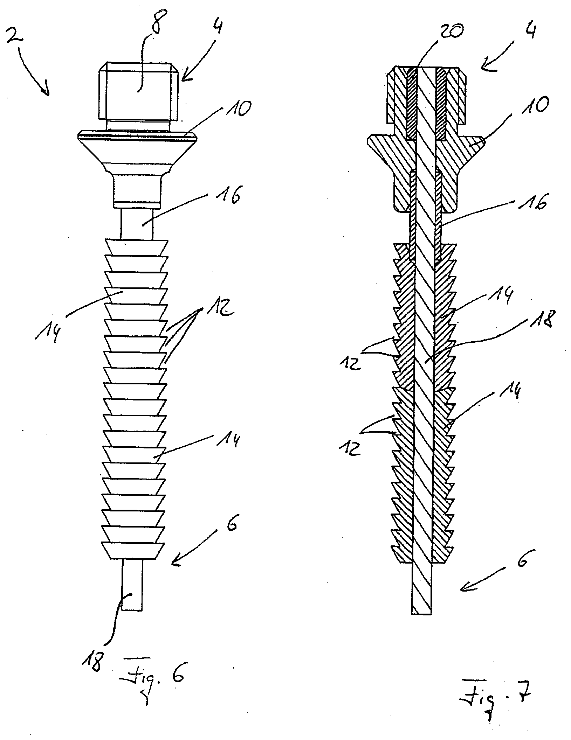

Figur 6 zeigt eine weitere Ausführungsform des Verbindungsstiftes 2. Er unterscheidet sich von der Darstellung in Figur 1 insbesondere durch die Form der Nuten 12. Diese sind nicht, wie in Figur 1, U-förmig ausgebildet, sondern weisen eine sägezahnartige Form auf. Dabei ist in Figur 6 zu erkennen, dass jede Nut 12 eine schräg verlaufende Flanke und eine horizontal verlaufende Flanke aufweist.

Figur 7 zeigt eine schematische Schnittdarstellung durch den Verbindungsstift 2 aus Figur 6. Auch diese Darstellung unterscheidet sich von der Darstellung in Figur 2 im Wesentlichen durch die Form der einzelnen Nuten 12. Wird der Verbindungsstift 2 gemäß den Figuren 6 und 7 in eine Aufnahmeeinrichtung 22 bzw. deren Aufnahme 24 eingeführt, greift er nicht in ein Ritzel ein, das durch das Einschieben des Verbindungsstiftes 2 gedreht wird, sondern gerät in Eingriff mit einem Zahnvorsprung oder ähnlichem, der in die einzelnen Nuten 12 hineinschnappt und so ein Herausziehen des Verbindungsstiftes 2 aus der Aufnahme 24, also einer Bewegung nach oben in den Figuren 6 und 7, entgegenwirkt.

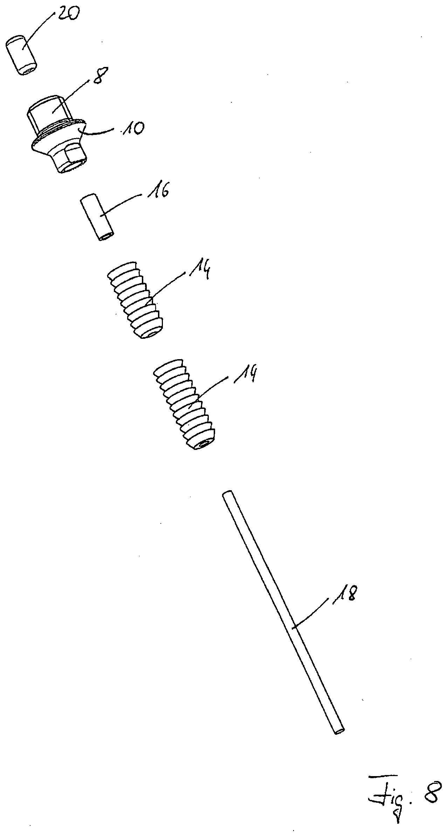

In Figur 8 ist eine Explosionsdarstellung des Verbindungsstiftes 2 aus den Figuren 6 und 7 dargestellt. Man erkennt, dass sich der Verbindungsstift 2 im Aufbau nicht von dem Verbindungsstift 2 aus den Figuren 1 bis 3 unterscheidet, sondern lediglich die Stiftsegmente 14 eine andere Art der Nuten 12 aufweisen.

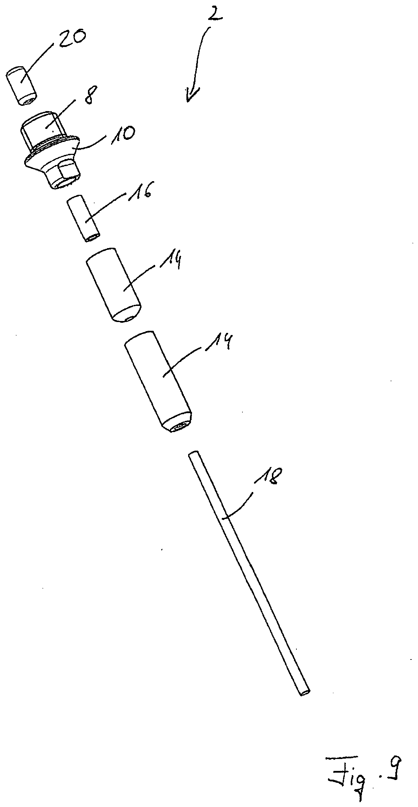

Figur 9 zeigt eine weitere Ausführungsform des Verbindungsstiftes 2 in einer Explosionsdarstellung. Auch dieser Verbindungsstift 2 unterscheidet sich im Aufbau nicht von den bisher gezeigten Verbindungsstiften 2. Der Unterschied liegt lediglich darin, dass die einzelnen Stiftsegmente 14 im in Figur 9 gezeigten Ausführungsbeispiel keine Nuten 12 aufweisen, sondern über eine glatte Mantelfläche verfügen. Wird ein derartiger Verbindungsstift 2 in die dafür vorgesehene Aufnahme 24 der Aufnahmeeinrichtung 22 eingeführt, können die Stiftsegmente 14 nicht mit einem Zahnelement oder einem Ritzel in Eingriff gebracht werden. In diesem Fall wird das Herausziehen des Verbindungsstiftes 2 aus der Aufnahme 24 durch einen Klemmmechanismus verhindert.

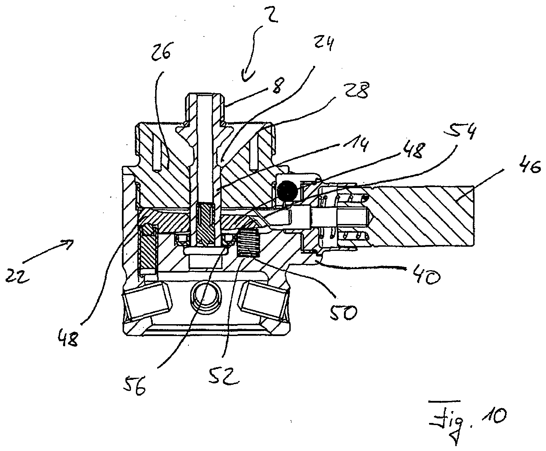

Dieser ist in Figur 10 in einer schematischen Schnittdarstellung durch das Verbindungssystem dargestellt. Man erkennt den Verbindungsstift 2, der in der Aufnahmeeinrichtung 22 angeordnet ist. Die Aufnahmeeinrichtung 22 weist wieder die Einführeinrichtung 26 sowie den Trichter 28 auf, um den Verbindungsstift 2 möglichst komfortabel und einfach in die Aufnahmeeinrichtung 22 bzw. deren Aufnahme 24 einführen zu können.

Anders als in der Darstellung der Figur 4 weisen die Stiftsegmente 14 im in Figur 10 gezeigten Ausführungsbeispiel eine glatte Außenfläche auf. Der Verbindungsstift 2 wird beim Einführen in die Aufnahme 24 durch eine in der Aufnahmeeinrichtung 22 angeordnete Klemmscheibe 48 hindurchgeführt. Diese weist eine zentrale Bohrung auf, durch die der Verbindungsstift 2 hindurchgeführt wird.

In Figur 10 ist dargestellt, dass die Klemmscheibe 48 in ihrem rechten Bereich auf einer Klemmfeder 50 gelagert ist. Bei der Klemmfeder 50 handelt es sich um eine Druckfeder, die in einer dafür vorgesehenen Ausnehmung 52 angeordnet ist. Die Klemmfeder 50 drückt den rechten Bereich der Klemmscheibe 48 nach oben, so dass es zu einer Verkantung des Verbindungsstiftes 2 in der zentralen Bohrung der Klemmscheibe 48 kommt. Dadurch wird ein Herausziehen des Verbindungsstiftes (2), in Figur 10 nach oben, sicher verhindert.

Soll der Verbindungsstift 2 hingegen weiter in die Aufnahme 24 eingeführt werden, wird dadurch auch eine Kraft auf die Klemmscheibe 48 aufgebracht, so dass die Klemmfeder 50 zusammengedrückt wird und die Verkantung so aufgehoben wird.

Um den Verbindungsstift 2 wieder aus der Aufnahme 24 zu entfernen, ist an der Verriegelungsplatte 40 das Bedienelement 46 angeordnet. Dieses weist an seinem im Inneren der Aufnahmeeinrichtung 22 zugewandten Ende eine erste Abschrägung 54 auf. Die erste Abschrägung 54 steht mit einer an der Klemmscheibe 48 vorgesehenen zweiten Abschrägung 56 in Kontakt. Wird nun das Bedienelement 46 in Richtung auf den in die Aufnahme 24 eingebrachten Verbindungsstift 2 bewegt, gleiten die erste Abschrägung 54 und die zweite Abschrägung 56 aufeinander ab, wodurch der rechte Bereich der Klemmscheibe 48 nach unten bewegt wird und so die Klemmfeder 50 zusammendrückt. Dadurch wird die Verkantung des Verbindungsstiftes 2 in der Klemmscheibe 48 aufgehoben, so dass der Verbindungsstift 2 aus der Aufnahme 24 entfernt werden kann.

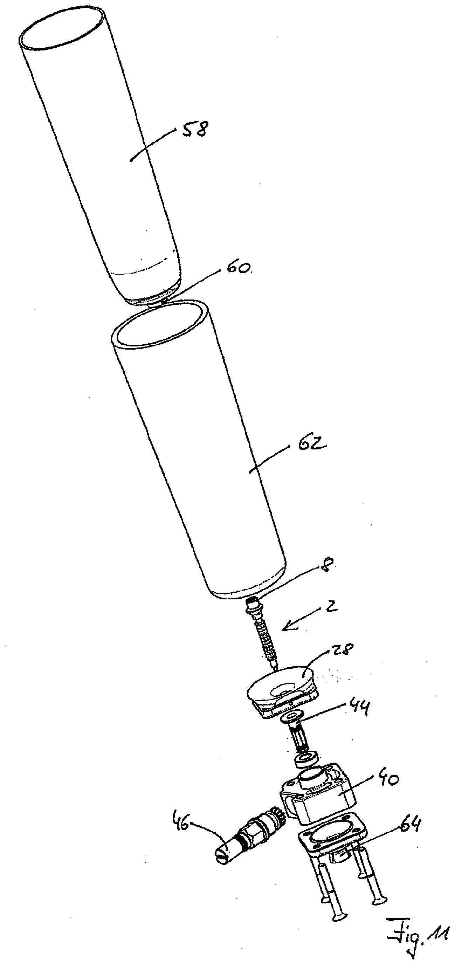

Figur 11 zeigt eine Explosionsdarstellung eines Prothesensystems gemäß einem Ausführungsbeispiel der Erfindung. Man erkennt einen Liner 58, an dessen unterem Ende sich eine Befestigungsvorrichtung 60 befindet, an der das Verbindungselement 8 des Verbindungsstiftes 2 angeordnet werden kann. Der Liner 48 wird in einem Prothesenschaft 62 angeordnet, an dessen unterem Ende die Aufnahmeeinrichtung 22 angeordnet ist, wie sie beispielsweise in Figur 5 dargestellt ist. Am unteren Ende der Verriegelungsplatte 40 wird über weitere Verbindungsmittel 64 die eigentliche künstliche Gliedmaße angeordnet.

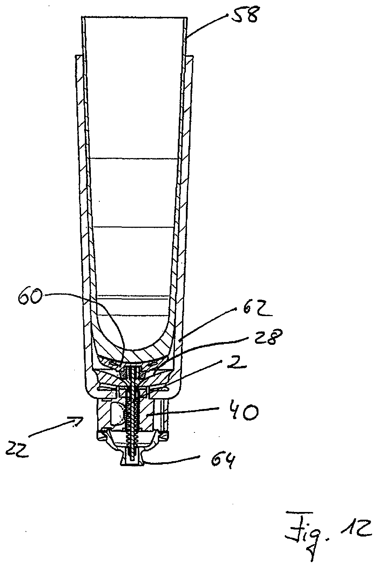

Figur 12 zeigt eine Schnittdarstellung durch das in Figur 11 in Form einer Explosionsdarstellung dargestellte Prothesensystem. Man erkennt den Liner 58, an dem sich die Befestigungsvorrichtung 60 für den Verbindungsstift 2 befindet. Der Liner 58 ist in dem Prothesenschaft 62 angeordnet, an dessen unterem Ende sich die Aufnahmeeinrichtung 22 befindet. Im in Figur 12 gezeigten Zustand ist der Verbindungsstift 2 in die Aufnahmeeinrichtung 22 bzw. deren Aufnahme 24 eingeführt.

**Bezugszeichenliste**

| | | | |
|---|---|---|---|
| 2 | Verbindungsstift | 48 | Klemmscheibe |
| 4 | Erstes Ende | 50 | Klemmfeder |
| 6 | Zweites Ende | 52 | Ausnehmung |
| 8 | Verbindungselement | 54 | Erste Abschrägung |
| 10 | Anschlag | 56 | Zweite Abschrägung |
| 12 | Nut | 58 | Liner |
| 14 | Stiftsegment | 60 | Befestigungsvorrichtung |
| 16 | Spannelement | 62 | Prothesenschaft |
| 18 | Flexibles Führungselement | 64 | Verbindungsmittel |
| 20 | Festlegung | | |
| 22 | Aufnahmeeinrichtung | | |
| 24 | Aufnahme | | |
| 26 | Einführeinrichtung | | |
| 28 | Trichter | | |
| 30 | Magnetisches Element | | |
| 32 | Ritzel | | |
| 34 | Zähne | | |
| 36 | Rasteinheit | | |
| 38 | Öffnung | | |
| 40 | Verriegelungsplatte | | |
| 42 | Zentralbohrung | | |
| 44 | Einpressbuchse | | |
| 46 | Bedienelement | | |

## Patentansprüche

1. Verbindungssystem eingerichtet zum lösbaren Verbinden eines Prothesenschaftes mit einem über einen Amputationsstumpf gezogenen Liner, das
- einen Verbindungsstift (2) und
- eine Aufnahmeeinrichtung (22) mit einer Aufnahme (24) umfasst, in die der Verbindungsstift (2) einführbar ist,
**dadurch gekennzeichnet, dass** der Verbindungsstift (2) wenigstens zwei starre Stiftsegmente (14) umfasst, die entlang einer Längsrichtung des Verbindungsstiftes (2) gegeneinander verlagerbar hintereinander angeordnet sind.

2. Verbindungssystem nach Anspruch 1, **dadurch gekennzeichnet, dass** die Stiftsegmente (14) auf ein flexibles Führungselement (18) aufgefädelt sind.

3. Verbindungssystem nach Anspruch 2, **dadurch gekennzeichnet, dass** der Verbindungsstift (2) ein erstes Ende (4) zum Anordnen an dem Liner und ein dem ersten Ende (4) entgegengesetztes zweites Ende (6) aufweist und die Stiftsegmente (14) durch ein Spannelement (16) in Richtung auf das zweite Ende (6) vorgespannt sind.

4. Verbindungssystem nach Anspruch 3, **dadurch gekennzeichnet, dass** das Spannelement (16) eine Feder, insbesondere eine Schraubenfeder, oder ein Elastomer, beispielsweise in Form eines Schlauches, umfasst.

5. Verbindungssystem nach Anspruch 3 oder 4, **dadurch gekennzeichnet, dass** das Spannelement (16) auf das flexible Führungselement (18) aufgefädelt ist.

6. Verbindungssystem nach Anspruch 5, **dadurch gekennzeichnet, dass** das Spannelement (16) zwischen den Stiftsegmenten (14) und dem ersten Ende (4) des Verbindungsstiftes (2) angeordnet ist.

7. Verbindungssystem nach einem der vorstehenden Ansprüche, **dadurch gekennzeichnet, dass** die Stiftsegmente (14) eine Mehrzahl von um die Längsrichtung umlaufenden Nuten (12) aufweisen, die ausgebildet sind, um in ein in der Aufnahmeeinrichtung (22) vorgesehenes Ritzel (32) einzugreifen, wobei das Ritzel (32) in eine Einführposition, in der der Verbindungsstift (2) in die Aufnahmeeinrichtung (22) einführbar ist, und in eine Freigabeposition bringbar ist, in der der Verbindungsstift (2) aus der Aufnahmeeinrichtung (22) entfernbar ist.

8. Verbindungssystem nach einem der vorstehenden Ansprüche, **dadurch gekennzeichnet, dass** die Aufnahmeeinrichtung (22) eine Einführeinrichtung (26) aufweist, durch die die Orientierung und/oder die Positionierung des Verbindungsstiftes (2) in der Aufnahme (24) vereinfacht wird.

9. Verbindungssystem nach Anspruch 8, **dadurch gekennzeichnet, dass** die Einführeinrichtung (26) wenigstens ein magnetisches Element (30) umfasst, und der Verbindungsstift (2) zumindest teilweise aus einem Material besteht, auf das durch das magnetische Element (30) eine Kraft ausgeübt wird.

10. Prothesensystem mit einem Liner und einem Prothesenschaft, wobei das Prothesensystem ein Verbindungssystem nach einem der vorstehenden Ansprüche umfasst, und der Verbindungsstift (2) an dem Liner und die Aufnahmeeinrichtung (22) an dem Prothesenschaft angeordnet sind.

## Claims

1. A connecting system arranged for detachably connecting a prosthesis shaft to a liner that has been pulled over a residual limb, said system including
- a connecting pin (2) and
- a receiving device (22) with a receiving means (24) into which the connecting pin can be inserted,
**characterized in that** the connecting pin (2) includes at least two rigid pin segments (14) which are arranged one behind another so as to be displaceable toward one another along a longitudinal direction of the connecting pin (2).

2. The connecting system as claimed in claim 1, **characterized in that** the pin segments (14) are threaded onto a flexible guide element (18).

3. The connecting system as claimed in claim 2, **characterized in that** the connecting pin (2) comprises a first end (4) for arranging on the liner and a second end (6) which is opposite the first end (4) and the pin segments (14) are prestressed by a tensioning element (16) in the direction of the second end (6).

4. The connecting system as claimed in claim 3, **characterized in that** the tensioning element (16) includes a spring, in particular a helical spring, or an elastomer, for example in the form of a hose.

5. The connecting system as claimed in claim 3 or 4, **characterized in that** the tensioning element (16) is threaded onto the flexible guide element (18).

6. The connecting system as claimed in claim 5, **characterized in that** the tensioning element (16) is arranged between the pin segments (14) and the first end (4) of the connecting pin (2).

7. The connecting system as claimed in one of the preceding claims, **characterized in that** the pin segments (14) comprise a plurality of grooves (12) which run around the longitudinal direction and are realized in order to engage in a pinion (32) which is provided in the receiving device (22), wherein the pinion (32) is movable into an insertion position in which the connecting pin (2) is insertable into the receiving device (22), and into a release position in which the connecting pin (2) is removable out of the receiving device (22).

8. The connecting system as claimed in one of the preceding claims, **characterized in that** the receiving device (22) comprises an insertion device (26) by means of which the orienting and/or the positioning of the connecting pin (2) in the receiving means (24) is simplified.

9. The connecting system as claimed in claim 8, **characterized in that** the insertion device (26) includes at least one magnetic element (30), and the connecting pin (2) consists at least in part of a material onto which a force is exerted as a result of the magnetic element (30).

10. A prosthesis system having a liner and a prosthesis shaft, wherein the prosthesis system includes a connecting system as claimed in one of the preceding claims, and the connecting pin (2) is arranged on the liner and the receiving device (22) is arranged on the prosthesis shaft.

## Revendications

1. Système de liaison conçu pour la liaison détachable d'une emboîture de prothèse à un doublage enfilé sur un moignon d'amputation, qui comprend
- une tige de liaison (2) et
- un dispositif de réception (22) pourvu d'un logement (24) dans lequel peut être introduite la tige de liaison (2),
**caractérisé en ce que** la tige de liaison (2) comprend au moins deux segments de tige rigides (14) qui sont agencés l'un derrière l'autre en étant mobiles l'un par rapport à l'autre le long d'une direction longitudinale de la tige de liaison (2).

2. Système de liaison selon la revendication 1, **caractérisé en ce que** les segments de tige (14) sont enfilés sur un élément de guidage flexible (18).

3. Système de liaison selon la revendication 2, **caractérisé en ce que** la tige de liaison (2) comprend une première extrémité (4) pour l'agencement sur le doublage et une seconde extrémité (6) opposée à la première extrémité (4), et les segments de tige (14) sont précontraints en direction de la seconde extrémité (6) par un élément de serrage (16).

4. Système de liaison selon la revendication 3, **caractérisé en ce que** l'élément de serrage (16) comprend un ressort, en particulier un ressort hélicoïdal, ou un élastomère, par exemple sous la forme d'un tuyau flexible.

5. Système de liaison selon la revendication 3 ou 4, **caractérisé en ce que** l'élément de serrage (16) est enfilé sur l'élément de guidage flexible (18).

6. Système de liaison selon la revendication 5, **caractérisé en ce que** l'élément de serrage (16) est agencé entre les segments de tige (14) et la première extrémité (4) de la tige de liaison (2).

7. Système de liaison selon l'une des revendications précédentes, **caractérisé en ce que** les segments de tige (14) présentent une pluralité de gorges (12) périphériques autour de la direction longitudinale, qui sont réalisées pour venir s'engager dans un pignon (32) prévu dans le dispositif de réception, le pignon (32) pouvant être amené dans une position d'introduction dans laquelle la tige de liaison (2) peut être introduite dans le dispositif de réception (22), et dans une position de libération dans laquelle la tige de liaison (2) peut être enlevée hors du dispositif de réception (22).

8. Système de liaison selon l'une des revendications précédentes, **caractérisé en ce que** le dispositif de réception (22) comprend un dispositif d'introduction (26) par lequel l'orientation et/ou le positionnement de la tige de liaison (2) dans le logement (24) est simplifié(e).

9. Système de liaison selon la revendication 8, **caractérisé en ce que** le dispositif d'introduction (26) comprend au moins un élément magnétique (30), et la tige de liaison (2) est constituée au moins partiellement en un matériau sur lequel une force est exercée par l'élément magnétique (30).

10. Système de prothèse comportant un doublage et une emboîture de prothèse, le système de prothèse comprenant un système de liaison selon l'une des revendications précédentes, et la tige de liaison (2) est agencée sur le doublage et le dispositif de réception (22) est agencé sur l'emboîture de prothèse.
